# EUROPEAN PATENT SPECIFICATION

(11) **EP 0 702 518 B1**
(45) Date of publication and mention of the grant of the patent: **02.04.2003**
(21) Application number: 94918203.4
(22) Date of filing: 02.06.1994
(51) Int. Cl.: A01N 43/42, A61K 31/47

(54) **TREATMENT OF VASCULAR DEGENERATIVE DISEASES BY MODULATION OF ENDOGENOUS NITRIC OXIDE PRODUCTION OR ACTIVITY**
BEHANDLUNG DEGENERATIVER GEFAESSERKRANKUNGEN DURCH MODULATION DER ENDOGENEN STICKOXIDPRODUKTION ODER-AKTIVITAET
TRAITEMENT DES MALADIES VASCULAIRES DEGENERATIVES PAR MODULATION DE L'ACTIVITE OU DE LA PRODUCTION D'OXYDE NITRIQUE ENDOGENE

(30) Priority: 11.06.1993 US 76312; 21.01.1994 US 184519
(43) Date of publication of application: 27.03.1996
(60) Divisional application: 00102776.2
(73) Proprietor: THE BOARD OF TRUSTEES OF THE LELAND STANFORD JUNIOR UNIVERSITY, Stanford, CA 94304 (US)
(72) Inventor: COOKE, John, P., Palo Alto, CA 94306 (US); DZAU, Victor, J., Los Altos Hills, CA 94022 (US); GIBBONS, Gary, H., Palo Alto, CA 94303 (US)
(74) Representative: Harrison, David Christopher
(86) International application number: US9406203
(87) International publication number: WO94028721

(56) References cited:
- EP-A- 0 441 119
- EP-A- 0 546 796
- WO-A-93/18156
- PALMER ET AL.: "Vascular endothelial cells synthesize nitric oxide from L-arginine " NATURE, vol. 333, 16 June 1988, pages 664-666, XP002062482
- PALMER ET AL.: "L-Arginine is the Physiological Precursor for the Formation of Nitric Oxide in Endothelium-Dependent Relaxation" BIOCHEM. BIOPHYS. RES. COMMUN., vol. 153, no. 3, 30 June 1988, pages 1251-1256, XP002062483
- L.J. IGNARRO: "Endothelium-derived Nitric Oxide: Actions and Properties" FASEB J., vol. 3, 1989, pages 31-36, XP002062484
- A. WALLACE: "Do Deficiencies of Endothelial Derived Relaxing Factor Contribute to Myocardial Stunning?" J. CARD. SURG., vol. 8[Suppl], 1993, pages 325-328, XP002062485
- MARIN ET AL.: "Role of Endothelium-formed Nitric Oxide on Vascular Responses" GEN. PHARMAC., vol. 21, no. 5, 1990, pages 575-587, XP002062486
- COOKE ET AL.: "Antiatherogenic Effects of L-Arginine in the Hypercholesterolemic Rabbit" J. CLIN. INVEST., vol. 90, 1992, pages 1168-1172, XP002063110
- CREAGER ET AL.: "L-Arginine Improves Endothelium-dependent Vasodilation in Hypercholesterolemic Humans" J. CLIN. INVEST., vol. 90, 1992, pages 1248-1253, XP002063111
- THE JOURNAL OF BIOLOGICAL CHEMISTRY, vol. 267, no. 21, 25 July 1992, pages 14519-14522, XP002063112
- YOUICHI NUNOKAWA ET AL.: "Cloning of Inducible Nitric Oxide Synthase in Rat Vascular Smooth Muscle Cells" BIOCHEM. BIOPHYS. RES. COMMUN., vol. 191, no. 1, 26 February 1993, pages 89-94, XP002063113
- NATURE, Volume 327, issued 21 May 1986, (London), ANDREWS et al., "Low-Density Lipoproteins Inhibit Endothelium-Depend Relaxation in Rabbit Aorta", pages 237-239.
- ARTERIOSCLEROSIS AND THROMBOSIS, Volume 11(2), issued March/April 1991, BATH et al., "Nitric Oxide and Prostacyclin Divergence of Inhibitory Effects of Monocyte Chemotexis and Adhesion to Endothelium in Vitro", pages 254-260.
- THE LANCET, Volume 338, issued 21/28 December 1991, DREXEL et al., "Correction of Endothelial Dysfunction in Coronary Microcirculation of Hypercholesterolaemic Patients", pages 1546-1550.
- JOURNAL OF CLINICAL INVESTIGATION, Volume 83, issued May 1989, GARG et al., "Nitric Oxide Generating Vasodilators and 8-Bromo-Ecyclic Guanosine Monophosphate Inhibit Mitogenesis and Proliferation of Cultured Rat Vascular Smooth Muscle Cells", pages 1774-1777.
- CIRCULATION RESEARCH, Volume 67, No. 6, issued December 1990, GIRERD et al., "L-Arginine Augments Endothelium-Dependent Vasodilation in Cholesterol-Fed Rats", pages 1301-1308.
- CIRCULATION RESEARCH, Volume 54, No. 6, issued June 1984, HEISTAD et al., "Augmented Response to Vasoconstrictor Stimuli in Hypercholesterolemic and Atherosclerotic Monkeys", pages 711-718.
- PROCEEDING FROM NATIONAL ACADEMY OF SCIENCE USA, Volume 88, issued June 1991, KUBES et al., "Nitric Oxide an Endogenous Modulator of Leukocyte Adhesion", pages 4651-4655.
- NATURE, Volume 344, issued 08 March 1990, (London), KUGIYAMA et al., "Impairment of Endothelium Dependent Arterial Relaxation by Lysolecithin in Modified Low-Density Lipoproteins", pages 160-162.
- JOURNAL CLINICAL INVESTIGATION, Volume 86, issued December 1990, MINOR JR. et al., "Diet-Induced Atherosclerosis Increase the Release of Nitrogen Oxides from Rabbit Aorta", pages 2109-2116.
- CIRCULATION RESEARCH, Volume 65, No. 6, issued December 1989, POHL et al., "EDRF Increases 6MP in Platelets During Passage through the Coronary Vascular Bed", pages 1798-1803.
- BRITISH JOURNAL PHARMACOLOGY, Volume 92, issue May 1987, RADOMSKI et al., "Comparitive Pharmacology of Endothelium-Derived Relaxing Factor Nitric Acid and Prostacylin in Platelets", pages 181-187.
- THE NEW ENGLAND JOURNAL OF MEDICINE, Volume 314, No. 8, issued 20 February 1986, ROSS, "The Pathogenesis of Atherosclerosis", pages 488-500.
- JOURNAL CLINICAL INVESTIGATION, Volume 87, issued April 1991, ROSSITCH et al., "L-Arginine Normalizes Endothelial Function Cerebral Vessles from Hypercholesterolemic Rabbits", pages 1293-1299
- CIRCULATION RESEARCH, Volume 65, No. 3, issued September 1989, STAMLER et al., "N-Acetyl Cysteine Potentiates Platelet Inhibition by Endothelium Derived Relaxin Factor", pages 789-795.
- CIRCULATION RESEARCH, Volume 83, No. 6, issued June 1991, TANNER et al., "Oxidized Low Density Lipoproteins Inhibit Relaxations of Porcine Coronary Arteries", pages 2012-2019.
- CIRCULATION RESEARCH, Volume 66, No. 1, issued January 1990, TOMITA et al., "Rapid and Reversible Inhibition by Low Density Lipoprotein of the Endothelium-Dependent Relaxation to Hemostatic Substances in Porcine Coronary Arteries", pages 18-27.
- CIRCULATION, Volume 81, No. 5, issued May 1990, WEIDINGER et al., "Persistent Dysfunction of Regenerated Endothelium After Balloon Angioplay of Rabbit Iliac Artery", pages 1667-1679.
- JOURNAL CLINICAL INVESTIGATOR, Volume 81, issued June 1988, YAMAMOTO et al., "Videmicroscopic Demonstration of Defective Cholinergic Arteriolar Vasodilation in Artherosclerotic Rabbit", pages 1752-1768.

## Description

This invention was supported in part by the United States Government under Grant 1KO7HCO2660 (NHLBI). The U.S. Government may have an interest in this application.

The field of this invention is the modulation of NO activity, which finds application in the treatment of vascular degenerative diseases, particularly atherosclerosis and restenosis.

Atherosclerosis and vascular thrombosis are a major cause of morbidity and mortality, leading to coronary artery disease, myocardial infarction, and stroke. Atherosclerosis begins with an alteration in the endothelium, which lines the blood vessels. The endothelial alteration results in adherence of monocytes, which penetrate the endothelial lining and take up residence in the subintimal space between the endothelium and the vascular smooth muscle of the blood vessels. The monocytes absorb increasing amounts of cholesterol (largely in the form of oxidized or modified low-density lipoprotein) to form foam cells. Oxidized low-density lipoprotein (LDL) cholesterol alters the endothelium, and the underlying foam cells distort and eventually may even rupture through the endothelium.

Platelets adhere to the area of endothelial disruption and release a number of growth factors, including platelet derived growth factor (PDGF). PDGF, which is also released by foam cells and altered endothelial cells, stimulates migration and proliferation of vascular smooth muscle cells into the lesion. These smooth muscle cells release extracellular matrix (collagen and elastin) and the lesion continues to expand. Macrophages in the lesion elaborate proteases, and the resulting cell damage creates a necrotic core filled with cellular debris and lipid. The lesion is then referred to as a "complex lesion." Rupture of this lesion can lead to thrombosis and occlusion of the blood vessel. In the case of a coronary artery, rupture of a complex lesion may precipitate a myocardial infarction, whereas in the case of a carotid artery, stroke may ensue.

One of the treatments that cardiologists and other interventionalists employ to reopen a blood vessel which is narrowed by plaque is balloon angioplasty (approximately 300,000 coronary and 100,000 peripheral angioplasties are performed annually). Although balloon angioplasty is successful in a high percentage of the cases in opening the vessel, it unfortunately denudes the endothelium and injures the vessel in the process. This damage causes the migration and proliferation of vascular smooth muscle cells of the blood vessel into the area of injury to form a lesion, known as myointimal hyperplasia or restenosis. This new lesion leads to a recurrence of symptoms within three to six months after the angioplasty in a significant proportion of patients (30-40%).

Because of their great prevalence and serious consequences, it is critically important to find therapies which can diminish the incidence of atherosclerosis, vascular thrombosis and restenosis. Ideally, such therapies would inhibit the pathological processes associated with atherosclerosis, thereby providing prophylaxis or retarding the progression of the degenerative process.

As briefly summarized above, these pathological, processes are extremely complex, involving a variety of different cells which undergo changes in their character, composition, and activity, as well as in the nature of the factors which they secrete and the receptors that are up- or down-regulated. A substance released by the endothelium, "endothelium derived relaxing factor" (EDRF), may play an important role in inhibiting these pathologic processes. EDRF is now known to be nitric oxide (NO) or a labile nitroso compound which liberates NO. (For purposes of the subject invention, unless otherwise indicated, nitric oxide (NO) shall intend nitric oxide or the labile precursor.) This substance has been reported to relax vascular smooth muscle, inhibit platelet aggregation, inhibit mitogenesis and proliferation of cultured vascular smooth muscle, and leukocyte adherence. NO may have other effects, either direct or indirect, on the various cells associated with vascular walls and degenerative diseases of the vessel.

### Relevant Literature

Girerd, et al. (1990) Circulation Research 67, 1301-1308 report that intravenous administration of L-arginine potentiates endothelium-dependent relaxation in the hind limb of cholesterol-fed rabbits. The authors conclude that synthesis of EDRF can be increased by L-arginine in hypercholesterolemia. Rossitch, et al. (1991) J. Clin. Invst. 87, 1295-1299 report that *in vitro* administration of L-arginine to basilar arteries of hypercholesterolemic rabbits reverses the impairment of endothelium-dependent vasodilation and reduces vasoconstriction. They conclude that the abnormal vascular responses in hypercholesterolemic animals is due to a reversible reduction in intracellular arginine availability for metabolism to nitric oxide.

Creager, et al. (1992) J. Clin. Invest. 90, 1248-1253, report that intravenous administration of L-arginine improves endothelium-derived NO-dependent vasodilation in hypercholesterolemic patients.

Janssens et al (1992) J. Biol. Chem. 267, 14519-14522, show that NO is synthesised in endothelial cells from L-arginine by NO synthase and report the cloning and expression a cDNA encoding human endothelial NO synthase.

Cooke, et al., "Endothelial Dysfunction in Hypercholesterolemia is Corrected by L-arginine," Endothelial Mechanisms of Vasomotor Control, eds. Drexler, Zeiher, Bassenge, and Just; Steinkopff Verlag Darmstadt, 1991, pp. 173-181, review the results of the earlier references and suggest, "If the result of these investigations may be extrapolated, exogenous administration of L-arginine (i.e., in the form of dietary supplements) might represent a therapeutic adjunct in the treatment and/or prevention of atherosclerosis."

Cooke, (1990) Current Opinion in Cardiology 5, 637-644 discusses the role of the endothelium in the atherosclerosis and restenosis, and the effect that these disorders have on endothelial function.

Cooke (1992) J. Clin. Invest. 90, 1168-1172, describe the effect of chronic administration of oral L-arginine in hypercholesterolemic animals on atherosclerosis. This is the first demonstration that oral L-arginine supplements can improve the release of NO from the vessel wall. The increase in NO release from the vessel wall was associated with a striking reduction in atherosclerosis in hypercholesterolemic animals. This is the first evidence to support the hypothesis that increasing NO production by the vessel wall inhibits the development of atherosclerosis.

Cooke and Tsao, (1992) Current Opinion in Cardiology 7, 799-804 describe the mechanism of the progression of atherosclerosis and suggest that inhibition of nitric oxide may disturb vascular homeostasis and contribute to atherogenesis.

Cooke and Santosa, (1993) In: Steroid Hormones and Dysfunctional Bleeding, AAAS Press, review the activities of EDRF in a variety of roles and suggest that reversibility of endothelial dysfunction may be affected by the stage of atherosclerosis. They conclude that EDRF is a potent vasodilator, plays a key role in modulating conduit and resistance vessel tone, has important effects on cell growth and interactions of circulatory blood cells with a vessel wall, and that disturbances of EDRF activity may initiate or contribute to septic shock, hypertension, vasospasm, toxemia and atherosclerosis.

Fitzpatrick, et al., American Journal of Physiology 265 (Heart Circ. Physiol. 34):H774-H778, 1993 report that wine and other grape products may have endothelium-dependent vasorelaxing activity, *in vitro.*

Other references which refer to activities attributed to NO or its precursor include: Pohl and Busse (1989) Circ. Res. 65:1798-1803; Radomski et al. (1987) Br. J. Pharmacol. 92:181-187; and Stamler et al. (1989) Circ. Res. 65:789-795; anti-platelet activity); Garg and Hassid (1989) J. Clin. Invest. 83:1774-1777; and Weidinger et al, (1990) Circulation 81:1667-1679; (NO activity in relation to vascular smooth muscle growth); Ross (1986) N. Engl. J. Med. 314:488-500; Bath et al. (1991) Arterioscler. Thromb. 11:254-260; Kubes et al. (1991) Proc. Natl. Acad. Sci. USA 89:6348-6352; Lefer et al. (1990) In: Endothelium-Derived Contracting Factors. Basel, S. Karger, pp.190-197; (NO activity in relation to leukocyte adhesion and migration); Heistad et al. (1984) Circ. Res. 43:711-718; Rossitch et al. (1991) J. Clin. Invest. 87:1295-1299; Yamamoto et al. (1988) ibid 81:1752-1758; Andrews et al. (1987) Nature 327:237-239; Tomita et al. (1990)Circ. Res. 66:18-27; Kugiyama et al. (1990) Nature 344:160-162; Mitchell et al. (1992) J. Vasc. Res. 29:169 (abst.); and Minor et al. (1990) J. Clin. Invest. 86:2109-2116; (NO activity in relation to hypercholesterolemia); Tanner et al. (1991) Circulation 83:2012-2020; Kuo et al. (1992) Circ. Res. 70:f465-476; Drexler et al. (1991) Lancet 338:1546-1550; and Nakanishi et al. (1992) In: Scientific Conference on Functional and Structural Mechanisms of Vascular Control, Snowbird, UT, p. 86 (abstr.); relation of L-arginine to NO-dependent vasodilation.

The invention provides a use for a composition in the manufacture of a medicament for use in the treatment of atherosclerosis and restenosis. The enhancement of endogenous nitric oxide or secondary messenger availability at a physiological site inhibits the progression of restenosis and atherosclerosis. As a prophylaxis or treatment for atherosclerotic susceptible hosts, the medicament is chronically administered at an effective dosage. For restenosis, the medicament may be administered for a limited period since this pathological process generally abates 3-6 months after the vascular injury (i.e. angioplasty or atherectomy). NO or secondary messenger availablity may be enhanced by having the composition include a gene coding NO synthetase in an expression vector for transfection of vascular cells whereby enhance expression of NO synthase in the vessel wall, particularly at the lesion site, results in the release of NO from the vessel wall or a reduction in the degradation of NO or the secondary messenger, cyclic guanosine monophosphate ("cGMP").

In the drawings :

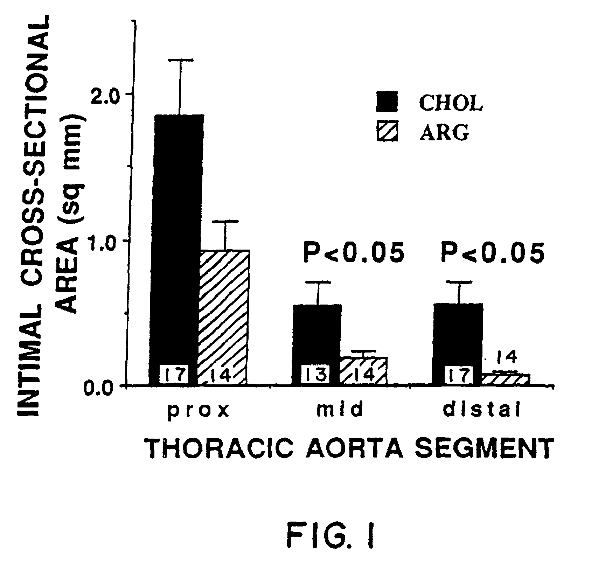

Fig. 1 is a bar diagram of histomorphometric studies of the effect of L-arginine on atheroscleortic plaque in hypercholesterolemic animals (See Ex. 1);
Fig. 2 is a nephelometric scan of the efffect of L-arginine diet supplement on platelet reactivity as evidenced by platelet aggregation initiated by adenosine diphosphate (See Ex. 2);
Fig. 3 is a bar diagram comparing the effect of L-arginine diet supplement on cell binding to aortic entdothelium of hypercholesterolemic animals (See Ex. 4); and
Fig. 4 is a bar diagram of morphometric measurements of intimal lesion thickness in EC-NOS treated animals in comparison to control vector or untreated injured vessels (See Ex. 6)

In accordance with the subject invention, common vascular degenerative diseases such as atherosclerosis, vascular thrombosis, and restenosis, are treated prophylactically and/or therapeutically by enhancing the physiological signal resulting from cellular response to endothelium-derived relaxing factor ("EDRF"), e.g. by maintaining an enhanced level of nitric oxide, its precursor, or a secondary messenger associated with the relaxation signal, e.g. cyclic guanosine monophosphate ("cGMP"), at the vessel wall in accordance with a predetermined regimen over an extended period of time. The enhanced level of nitric oxide (which is intended to include any precursor of nitric oxide which results in such enhanced level) can be achieved by modulating the activity, synthesis or concentration of any of the components associated with the formation of nitric oxide in the nitric oxide synthetic pathway, or inhibiting the rate of degradation of nitric oxide, its precursors, or the secondary messengers associated with the relaxation signal, by enhancing the amount of nitric oxide synthetase present in the vessel wall, particularly at the site of lesions. This can be done by local administration to the lesion site or systemically into the vascular system.

Cells are genetically engineered to provide for constitutive or inducible expression of one or more genes, which will provide for the desired relaxation response, by expressing NO synthase, or other enzymes or proteins which can be secreted and act extracellularly. Thus, expression vectors (viral or plasmid) may be prepared which contain the appropriate gene(s) and which can be introduced into host cells which will then produce high concentrations of nitric oxide or other intermediate in the relaxation pathway. These cells may be introduced at the lesion site or at another site in the host, where the expression will induce the appropriate response as to relaxation, proliferation, etc. The NO synthase or cells expressing the NO synthase may be present as depots by encapsulation and positioning at the site of interest. For example, porous stents may be produced which encapsulate the enzyme or cells to protect the enzyme from degradation and removal or, for the cells, to protect the cells from the immune system.

Cultured cells can be transfected with expression vectors containing the NO synthase or other gene ex-vivo and then introduced into the vessel wall, using various intra-arterial or intra-venous catheter delivery systems. Alternatively, techniques of in vivo gene transfer can be employed to transfect vascular cells within the intact vessel in vivo. The gene(s) can be expressed at high constitutive levels or can be linked to an inducible promoter (which may have tissue specificity) to allow for regulation of expression.

DNA constructs are prepared, where the appropriate gene, e.g. a NO synthase gene, is joined to an appropriate promoter, either with its native termination region or a different termination region, which may provide for enhanced stability of the messenger RNA. Constitutive promoters of particular interest will come from viruses, such as Simian virus, papilloma virus, adenovirus, HIV, Rous sarcoma virus, cytomegalovirus or the like, where the promoters include promoters for early or late genes, or long terminal repeats. Endogenous promoters may include the β-actin promoter, or cell-type specific promoters.

A construct is prepared in accordance with conventional techniques, the various DNA fragments being introduced into an appropriate plasmid or viral vector, normally a vector capable of replication in a bacterial and/or eucaryotic host. Normally, the vector will include a marker, which allows for selection of cells carrying the vector, e.g. antibiotic resistance. The vector will normally also include an origin which is functional in the host for replication. Other functional elements may also be present in the vector.

Once the vector has been prepared and replicated, it may then be used for introduction into host cells. The plasmid vector construct may be further modified by being joined to viral elements which allow for ease of transfection, may provide a marker for selection, e.g. antibiotic resistance, or other functional elements. Introduction of the plasmid vector construct into the host cells may be achieved by calcium phosphate precipitated DNA, transfection, electroporation, fusion, lipofection, viral capsid-mediated transfer, or the like. Alternatively, the expression construct within viral vectors may be introduced by standard infection techniques. For somatic cell gene therapy, autologous cells will generally be employed, although in some instances allogeneic cells or recombinantly modified cells may be employed. Usually the cells employed for genetic modification will be mature endothelial or vascular smooth muscle cells. Occasionally, the cells employed for genetic modification will be progenitor cells, particularly early progenitor cells. For example, myoblasts may be employed for muscle cells or hematopoietic stem cells or high proliferative potential cells may be employed for lymphoid and/or myelomonocytic cells.

Depending upon the nature of the cells, they may be injected into tissue of the same or different cellular nature, they may be injected into the vascular system, where they may remain as mobile cells or home to a particular site (i.e. the lesion). For the NO synthase gene, the number of cells which are administered will depend upon the nature of the cells, the level of production of the NO synthase, the desired level of NO synthase in the host vascular system, at the lesion site, or the like, whether the enhanced level of NO synthase is the only treatment or is used in conjunction with other components of the nitric oxide synthetic pathway, and the like. Therefore, the particular number of cells to be employed will be determined empirically in accordance with the requirements of the particular patient.

These cells may also be introduced into the circulation by first growing them on the surface of standard vascular graft material (i.e. Dacron or polytetrafluoroethylene grafts) or other synthetic vascular conduits or vascular bioprostheses. In this way, the cells may be introduced into the host by introducing the vascular graft seeded with the cells.

Alternatively, one may use viral vectors, which are capable of infecting cells *in vivo*, such as adenovirus or retroviruses. In this case, the viral vector containing the NO synthase gene or other gene involved with the relaxation pathway will be administered directly to the site of interest, where it will enter into a number of cells and become integrated into the cell genome. Thus, one can titer the desired level of nitric oxide synthase which is secreted or other protein which is expressed, by providing for one or more administrations of the virus, thus incrementally increasing the amount of synthase which is secreted or other protein which is produced.

Alternatively, one may use modified liposomes as a vehicle for endovascular administration of the vector containing the NO synthase or other gene. One such modified liposome technique involves mixing the liposomes with the vector containing NO synthase. Once the gene expression construct-containing vector is incorporated into the liposome, the liposomes are coated with a protein (e.g. the viral coat protein of the Hemagglutinating Virus of Japan) that increases the affinity of the liposome for the vessel wall.

In some situations, the NO synthase or other gene in the relaxation pathway may be co-transfected with an artificial gene encoding an arginine rich polypeptide susceptible to proteolytic cleavage as an intracellular source of L-arginine. In other situations, the NO synthase or other gene may be co-transfected with the superoxide dismutase gene, so as to inhibit the degradation of the nitric oxide.

The following discussion and examples are offered by way of illustration and not by way of limitation.

### EXPERIMENTAL

### Anti-atherogenic effects of oral arginine:

**Study design:** (See, Cooke et al., 1992, *supra*) Male New Zealand white rabbits (n = 49) were assigned to one of three treatment groups: 10 were fed with normal rabbit chow for ten weeks (Control); 19 received chow enriched with 1% cholesterol (Chol); and 20 received a 1% cholesterol diet supplemented with 2.5% L-arginine hydrochloride in the drinking water (Arg.). Following ten weeks of the dietary intervention, animals were lightly sedated and the central ear artery cannulated for measurement of intra-arterial blood pressure, followed by collection of blood samples for serum chemistries and plasma arginine. Subsequently the animals were sacrificed and the left main coronary artery and the thoracic aorta were harvested for studies of vascular reactivity and histomorphometry. In some animals, blood was collected for studies of platelet and monocyte reactivity.

**Results:** Biochemical and physiological measurements. Hypercholesterolemic animals maintained on oral L-arginine supplementation (Arg) experienced a twofold elevation in plasma arginine levels in comparison to animals on a normal (Control) or 1% cholesterol (Chol) diet alone; the elevation in plasma arginine was maintained throughout the course of the study. Serum cholesterol measurements were elevated equally in both groups receiving the 1 % cholesterol diet [50 ± 6 vs. 1629 ± 422 vs. 1852 ± 356 mg/dl respectively for Control (=10), Chol (=13), and Arg (=14)]. There were no significant differences in hemodynamic measurements between groups.

**Organ chamber studies of isolated vessels :** For NO-independent responses, there were no differences between the treatment groups in maximal response or sensitivity to norepinephrine (a vasoconstrictor), or to nitroglycerin (a nitrovasodilator). By contrast, NO-dependent relaxations were attenuated in vessels harvested from hypercholesterolemic animals with a reduction in the maximal response to acetylcholine, and a reduction in sensitivity to calcium ionophore. In comparison, vessels harvested from hypercholesterolemic animals receiving L-arginine supplementation had improved NO-dependent relaxation. Similarly, sensitivity of vessels to calcium ionophore A23187 was greater in the Arg group. In a separate study, the effect of chronic arginine supplementation to improve NO-dependent relaxation was confirmed in the hypercholesterolemic rabbit abdominal aorta.

**Histomorphometric studies (planimetry of EVG-stained sections):** A blinded histomorphometric analysis revealed that medial cross-sectional areas of thoracic aortae were not different between the groups. By contrast, the intimal cross-sectional area (i.e. amount of atherosclerotic plaque) of vessels from hypercholesterolemic animals receiving L-arginine supplementation was reduced in comparison to those from animals receiving cholesterol diet alone. In the Arg animals the reduction in the intimal lesion was most pronounced in the ascending thoracic aorta and left main coronary artery. In the left main coronary artery of hypercholesterolemic animals receiving arginine, essentially no atherosclerotic plaque developed.

**Changes in lesion surface area:** In a second series of studies, the extent of the thoracic aorta involved by lesions was examined. In hypercholesterolemic rabbits receiving vehicle (n=6) or L-arginine supplement (n = 6), thoracic aortae (from left subclavian artery to diaphragm) were harvested after ten weeks of treatment, bisected longitudinally, and stained with oil-red O. Vessels were photographed and vessel and lesion surface area determined by planimetry. Approximately 40% of the total surface area was covered with plaque in thoracic aortae from hypercholesterolemic animals receiving vehicle, whereas in thoracic aortae from arginine-treated hypercholesterolemic animals, less than 10% of the surface area was covered with plaque.

To summarize, dietary arginine supplementation increases plasma arginine levels, but does not alter serum cholesterol. This is associated with significant improvement in NO-dependent vasodilation as judged by bioassay. Finally, the improvement in NO-dependent vasodilation is associated with reduction in thickness and area of the lesions in vessels from hypercholesterolemic animals.

### Inhibition of platelet aggregation by oral L-arginine:

The effect of L-arginine supplementation on platelet reactivity in rabbits that had normal chow (Control; n = 6), a 1% cholesterol diet (Chol; n = 5), or a 1 % cholesterol diet supplemented with oral arginine (Arg; n = 6), as detailed above, was examined. Arterial blood obtained after central ear artery cannulation was anticoagulated with 13mM sodium citrate. Platelet-rich suspension was prepared by washing platelets in calcium-free Krebs-Henseleit solution and resuspending them in Tyrode's solution with albumin. The platelet count was adjusted at 2.5 x 10⁴ platelets/µl by addition of platelet-poor plasma. Aggregation was initiated by addition of adenosine diphosphate and monitored by standard nephelometric techniques. In platelets derived from Chol animals, aggregation was not different in rate or maximum extent in comparison to platelets from Control animals (A). By contrast, aggregation of platelets from Arg animals was reduced by 50% (B).

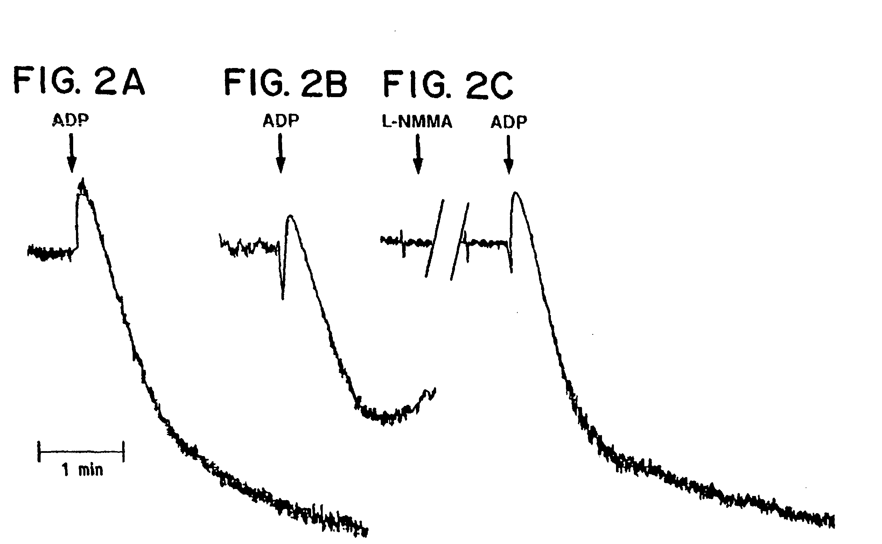

This reduction in platelet aggregation was associated with a twofold greater cGMP content in aggregated platelets from arginine-treated animals. The reduction of platelet reactivity could be reversed by administration of N-methylarginine (10⁻⁴ M) in vitro (C) (Fig. 2.) Therefore, the anti-platelet effect of chronic oral arginine administration can be credited to an increased synthesis of endogenous NO. Furthermore, NO synthesis may be induced in both the platelets and the endothelium.

### Inhibition of monocyte adherence:

A. **Functional Binding Assay:** To determine if oral arginine supplementation affects monocyte adherence, blood was collected from rabbits fed normal chow (=6), a 1% cholesterol diet (=6), or a 1 % cholesterol diet supplemented with L-arginine (=6), as described above. Mononuclear cells were purified from blood by Ficoll-paque density gradient centrifugation. In these preliminary studies, adhesion was examined of blood leukocytes to a transformed endothelial cell line, bEnd3 (mouse brain-derived polyoma middle T antigen transformed endothelial cells). The Bend3 cells display the morphology of endothelial cells, and like human endothelial cells are capable of uptake of acetylated low-density lipoprotein and express adhesion molecules in a cytokine-regulatable fashion. Cultured cells were grown to confluence in 0.5 cm² Lab-Tek chamber slides (MilesScientific) and treated with control medium or with LPS (1 mg/ml) or TNFa (25 U/ml) for 18 hours. Cultures were washed with fresh assay buffer, and low, medium, or high concentrations of leukocytes (0.75, 1.5, or 3 x 10⁵ cells/ml, respectively) were added per well. Following a 30-minute incubation on a rocking platform at room temperature to allow binding, the slides were inverted and immersed in buffer containing 2% (v/v) glutaraldehyde, such that non-adherent cells were lost and adherent cells were fixed to the monolayer. The adherent mononuclear cells were enumerated using video-light microscopy.

Monocytes from hypercholesterolemic animals (Chol) exhibited greater adherence, consistent with observation by others, that monocytes from hypercholesterolemic cats or humans exhibit greater adherence to cultured endothelial cells. (deGruijter, et al. (1991) Metabol. Clin. Exp. 40, 1119-1121; Fan, et al. (1991) Virchows Arch. B Cell Pathol. 61, 19-27).

In comparison to monocytes derived from vehicle-treated hypercholesterolemic animals (Chol), those from arginine-treated hypercholesterolemic animals (Arg) were much less adherent. This data shows that the arginine treatment inhibits adhesion of monocytes to the endothelium, which is the first observable event in atherogenesis.

### Dietary L-Arginine Inhibits the Enhanced Endothelial-Monocyte Interaction In Hypercholesterolemia

The earliest observable abnormality of the vessel wall in hypercholesterolemic animals is enhanced monocyte adherence to the endothelium, which occurs within one week of a high cholesterol diet. This event is thought to be mediated by the surface expression of endothelial adhesion molecules and chemotactic proteins induced by hypercholesterolemia.

Another endothelial alteration that occurs in parallel is a reduced activity of nitric oxide (i.e., NO), derived from metabolism of L-arginine. As shown above chronic dietary supplementation with L-arginine restores NO-dependent vasodilatation in hypercholesterolemic rabbits, and that this improvement in NO activity is associated with a striking anti-atherogenic effect. In the following study was tested the hypothesis that the anti-atherogenic effect of dietary arginine was mediated by endothelial derived NO which inhibits monocyte-endotheliat cell interaction.

### Methods

*Animals*. Male New Zealand White rabbits were pair fed, receiving one of the following dietary interventions for 2 weeks: normal rabbit chow (Cont, n = 7); rabbit chow enriched with 1 % cholesterol (Chol, n = 7); or 1 % cholesterol chow supplemented with 2.25% L-arginine HCI in the drinking water (Arg, n = 7) *ad libitum* throughout the course of the study. In a second series of studies designed to further explore the role of endogenous NO on monocyte-endothelial cell interaction, another group of animals were pair fed, receiving a normal rabbit diet supplemented with either vehicle control (N = 5) or the NO synthase antagonist, nitro-L-arginine (L-NA, 10 mg/100 ml; n = 5), administered in the drinking water *ad libitum* throughout the course of the study (for an average daily dose of 13.5 mg/kg/day). In a third series of experiments animals received a normal diet and either vehicle (n=4), L-NA (13.5 mg/kg/day; n = 4), or L-NA and hydralazine (n = 4) added to the drinking water for two weeks. At this dose, hydralazine (5 mg/kg/day) reversed the increase in blood pressure induced by L-NA. One day before sacrifice (after 2 weeks of dietary intervention), animals were lightly sedated and the central ear artery was cannulated for collection of blood samples.

*Mononuclear cell culture and isolation*. Murine monocytoid cells, WEHI 78/24 cells were grown in Dulbecco's Modified Eagle's Medium supplemented 10% fetal calf serum (vol/vol) and were kept in an atmosphere of 5% CO₂/95% air. Prior to binding studies, mononuclear cells were fluorescently labeled with TRITC (3 µg/ml). To confirm the results using WEHI cells, in some studies binding studies were performed in parallel using rabbit mononuclear cells. Mononuclear cells were isolated from fresh whole blood of Control rabbits before sacrifice.

*Preparation of aortic endothelium and binding assay*. After 2 weeks of the dietery intervention, the thoracic aortae were removed and placed in cold, oxygenated saline. A 15 mm segment of thoracic aorta was excised from a point immediately distal to the left subclavian artery to the midthoracic aorta. The segments were then carefully opened longitudinally and placed into culture dishes containing HBSS medium. Aortic strips were fixed to the culture dish using 25 gauge needles so as to expose the endothelial surface to the medium. Culture dishes were then placed on a rocking platform at room temperature.

After 10 minutes the HBSS medium was replaced by binding medium containing WEHI cells. The aortic strips were incubated with the mononuclear cells for 30 minutes. The medium was then replaced by fresh binding medium without cells to remove non-adherent cells. The aortic segments were then removed and placed on a glass slide, and adherent cells counted under epifluorescent microscopy from at least 30 sites on each segment.

### Results

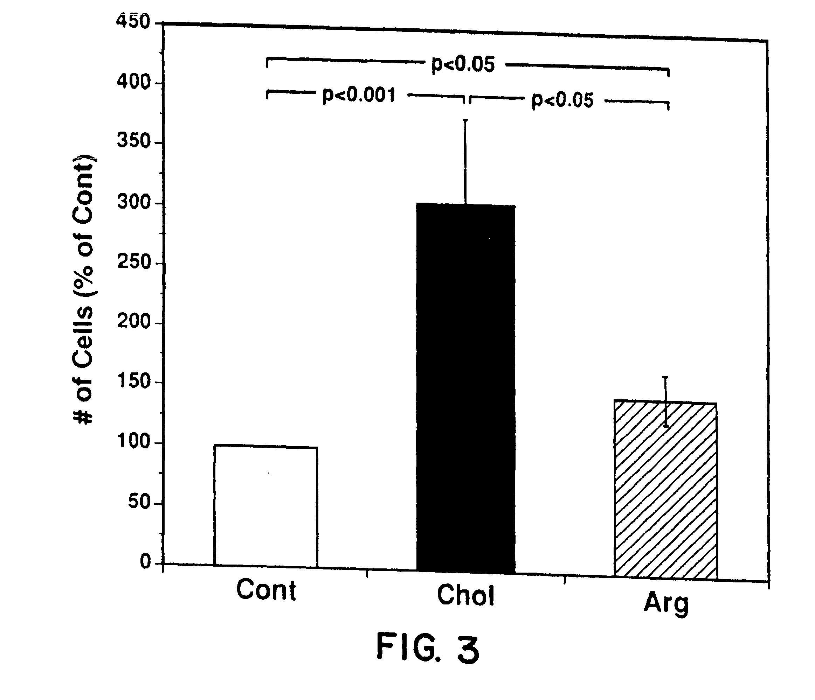

*Monocyte adhesion to rabbit aortic endothelium*. Exposure of WEHI 78/24 cells to normal rabbit aortic endothelium results in a minimal cell binding in this *ex vivo* adhesion assay. However, when WEHI cells were incubated with aortic endothelium from hypercholesterolemic animals (Chol; n = 7), cell binding was enhanced 3-fold in comparison to Cont (n = 7). The increased cell binding manifested by aortic endothelium of hypercholesterolemic animals was significantly attenuated by L-arginine supplementation (n = 7). (Fig. 3) Similar results were achieved when adhesion assays were performed in parallel with mononuclear cells that were freshly isolated from Cont animals (n = 2) in each of the three

*Effect of chronic NO synthase inhibition on endothelial adhesiveness*. To further investigate the role of endothelium-derived NO in modulating endothelial-monocyte interaction, an additional series of binding studies were performed using thoracic aorta from animals that received regular chow supplemented with vehicle (n = 5) or the NO synthase inhibitor, L-NA (n = 5). The adhesion of WEHI cells was markedly increased when incubated with aortic endothelium from L-NA animals compared to control endothelium. This effect could not be attributed to hypertension caused by L-NA since concomittant administration of hydratazine normalized blood pressure but did not reverse the augmentation of cell binding induced by L-NA.

In a separate series of studies it was confirmed that chronic administration of L-NA (the inhibitor of NO synthase) significantly inhibited generation and release of NO from the vessel wall (as measured by chemiluminescence), compared to vessels from animals treated with vehicle or arginine.

The salient findings of this investigation are: 1) monocyte binding to the endothelium *ex vivo* is increased in vessels from hypercholesterolemic animals; 2) this increase in monocyte binding is attenuated in hypercholesterolemic animals treated chronically with the NO precursor L-arginine; 3) monocyte binding to the endothelium is increased in vessels from normocholesterolemic animals treated with the NO synthase antagonist L-nitro-arginine; and 4) this effect of NO synthase antagonism was not reversed by administration of hydralazine in doses sufficient to normalize blood pressure. These findings are consistent with the hypothesis that NO inhibits monocyte-endothelial cell interaction.

To conclude, an *ex vivo* model of monocyte binding has been used to study the increase in endothelial adhesiveness induced by hypercholesterolemia. Attenuation of endothelial adhesiveness is attenuated by oral administration of the NO precursor L-arginine is shown. Conversely, inhibition of NO synthase activity by oral administration of nitro-arginine strikingly increases endothelial affinity for monocytes *ex vitro*. The data are consistent with NO being an endogenous anti-atherogenic molecule.

### Example 1. Effect of NO synthase expression on proliferation of vascular smooth muscle cells:

Cultured rat aortic vascular smooth muscle cells under confluent quiescent conditions were studied. An efficient viral coat protein-mediated DNA transfer method was employed to transfect the cells with the NO synthase gene driven by the β-actin promoter and CMV enhancer. This resulted in increased NO synthase activity (as measured by the arginine-to-citrulline conversion assay) in comparison to control vector transfected cells. Transfection of the NO synthase gene completely abolished serum-stimulated DNA synthesis compared to control vector transfection. These results indicated that increased expression of NO synthase (associated with increased production of NO) inhibits excessive proliferation of vascular smooth muscle cells. This inhibition can be correlated with treatment of atherosclerosis and restenosis.

### Example 2. Gene Therapy Using NO Synthase cDNA Prevents Restenosis

The study in Example 1 indicated that NO inhibits proliferation of vascular smooth muscle cells. In atherogenesis and restenosis, excessive proliferation of vascular smooth muscle cells contributes to lesion formation. Injury to the endothelium in atherosclerosis and after catheter interventions apparently reduces or removes the salutory influence of NO. The following study shows that delivery of the gene for NO synthase to the vessel wall inhibits lesion formation.

A plasmid construct encoding the cDNA of endothelial-type NO synthase (EC-NOS) was synthesized. A full length cDNA encoding for EC-NOS was inserted into the EcoRI site of the pUCcaggs expression vector. Balloon angioplasties of the carotid artery in Sprague-Dawley rats were performed and HVJ-liposomes with plasmids encoding EC-NOS cDNA infused, or plasmids lacking EC-NOS cDNA (control vector) infused. After 4 days to 2 weeks, the rats were sacrificed and the carotid arteries harvested for: 1) histomorphometry; 2) measurement of DNA synthesis; and 3) *ex vivo* determination of NO synthesis and release by bioassay and by chemiluminescence.

### Results

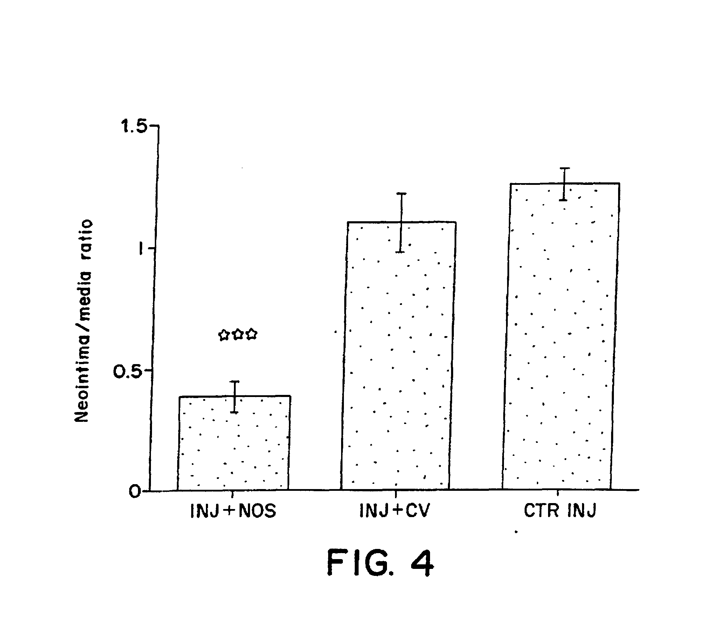

Morphometric measurements 2 weeks after injury revealed a significant (68%) reduction of intimal lesion thickness in EC-NOS treated (Inj + NOS) in comparison to control vector treated (Inj + CV) or untreated (Ctr Inj) injured vessels. (Fig. 4) Measurements of DNA synthesis were performed 4 days after injury using bromodeoxyuridine. EC-NOS transfection significantly limited bromodeoxyuridine incorporation (by 25%) in comparison to control vector treated or untreated injured vessels. Vessel segments were studied *ex vivo* using organ chamber technique to bioassay for NO release. Calcium ionophore increases intracellular calcium and activates NO synthase to produce NO. Calcium ionophore induced relaxations in injured carotid arteries transfected with control vector that were only 15% of uninjured vessels. Injured arteries that had been transfected with EC-NOS relaxed to a much greater degree, approximately 50% of that observed in uninjured vessels. Direct measurement of NO (by chemiluminescence) released into the medium revealed that NO released by injured tissues (transfected with the control vector) was only 20% of that released by normal uninjured tissues. By contrast, injured tissues transfected with EC-NOS released more NO (about 75% of normal).

To conclude, balloon angioplasty of the rat carotid artery removes the endothelial source of NO, induces excessive vascular smooth muscle DNA synthesis and proliferation, resulting in an intimal lesion (restenosis). Transfection of the vessel with EC-NOS at the time of balloon injury partially restores NO production by the vessel, and this is associated with reduced DNA synthesis and vascular smooth muscle proliferation, thereby reducing lesion formation. These results are consistent with the conclusion that NO is an endogenous anti-atherogenic molecule.

### Comparative Example. Exclusion of the Effect of Enhanced Nitrogen or Caloric Balance as Causing the Observed Results:

To exclude an effect of L-arginine on nitrogen or caloric balance as the cause of these results, six animals received 1% cholesterol diet supplemented by additional methionine to increase the dietary methionine six-fold. At ten weeks animals were sacrificed for studies of platelet and vascular reactivity, and histomorphometry. Endothelium-dependent relaxation, platelet aggregation and intimal thickness were not different from those of animals fed 1 % cholesterol diet alone. These results reveal that another amino acid, methionine (which is not a precursor of NO) does not mimic the effect of the amino acid L-arginine. Therefore it seems likely that the effect of L-arginine is due to its metabolism to nitric oxide, rather than some other effect of amino acid administration (i.e. change in nitrogen or caloric balance).

It is evident from the above results, that by enhancing the nitric oxide levels, by means of nitric oxide precursor compounds or other compounds in the nitric oxide pathway, substantial benefits will ensue to patients with vascular degenerative diseases. This treatment will diminish the formation of atherosclerotic plaque and restenosis, by inhibiting adhesion of monocytes and platelets, and by reducing the proliferation of vascular smooth muscle cells.

Thus we have shown that, one may use genetic engineering to introduce a gene associated with a component in the synthetic pathway for production of nitric oxide, e.g. nitric oxide synthase, where the enhanced production of such compounds will have the effect of driving the equilibrium to an enhanced production of nitric oxide. Thus, the subject invention provides a plurality of pathways to enhance the synthesis or action of nitric oxide, or reduce the degradation of nitric oxide, thereby increasing the effect of endogenous nitric oxide to prevent the formation of vascular lesions and to inhibit restenosis.

## Claims

1. The use of a composition in the preparation of a medicament for the inhibition of atherosclerosis or restenosis in the cardiovascular system of a mammalian host, said composition comprising:
a genetic construct comprising a gene encoding a nitric oxide synthetase in an expression vector for transfection of vascular cells;
wherein said nitric oxide synthetase is expressed by said vascular cells at a site of a lesion to enhance the level of nitric oxide and inhibit myointimal hyperplasia.

2. The use of claim 1 wherein said nitric oxide synthetase is endothelial cell nitric oxide synthetase.

3. The use according to any one of claims 1 to 2 wherein said medicament is formulated to be introduced into said host as a vascular graft seeded with said vascular cells.

4. The use according to any one of claims 1 to 3 wherein said vascular cells are endothelial cells.

5. The use according to any one of claims 1 to 3 wherein said vascular cells are vascular smooth muscle cells.

6. The use according to any one of claims 1 to 5 wherein said preparation of a medicament comprises mixing said genetic construct with a liposome.

7. The use according to any one of claims 1 to 6 wherein said vector is a plasmid vector.

8. The use according to any one of claims 1 to 6 wherein said vector is a viral vector.

9. The use according to any one of claims 1 to 8 wherein said myointimal hyperplasia follows balloon angioplasty.

## Patentansprüche

1. Verwendung einer Zusammensetzung zur Herstellung eines Medikaments zum Hemmen von Atherosklerose oder Restenose im Herz-Kreislauf-System eines Säugetierwirts, wobei die Zusammensetzung Folgendes umfasst:
ein genetisches Konstrukt, das ein Gen umfasst, das für eine Stickstoffoxid-Synthetase kodiert, in einem Expressionsvektor zur Transfektion von Gefäßzellen;
worin die Stickstoffoxid-Synthetase von den Gefäßzellen an einer Stelle einer Läsion exprimiert wird, um die Stickstoffoxidmenge zu erhöhen und myointimale Hyperplasie zu hemmen.

2. Verwendung nach Anspruch 1, worin die Stickstoffoxid-Synthetase Endothelzellen-Stickstoffoxid-Synthetase ist.

3. Verwendung nach einem der Ansprüche 1 bis 2, worin das Medikament dazu formuliert ist, in den Wirt als Gefäßtransplantat eingeführt zu werden, das mit den Gefäßzellen versetzt ist.

4. Verwendung nach einem der Ansprüche 1 bis 3, worin die Gefäßzellen Endothelzellen sind.

5. Verwendung nach einem der Ansprüche 1 bis 3, worin die Gefäßzellen Gefäß-Glattmuskelzellen sind.

6. Verwendung nach einem der Ansprüche 1 bis 5, worin die Herstellung eines Medikaments das Vermischen des genetischen Konstrukts mit einem Liposom umfasst.

7. Verwendung nach einem der Ansprüche 1 bis 6, worin der Vektor ein Plasmidvektor ist.

8. Verwendung nach einem der Ansprüche 1 bis 6, worin der Vektor ein viraler Vektor ist.

9. Verwendung nach einem der Ansprüche 1 bis 8, worin die myointimale Hyperplasie eine Folge Ballon-Angioplastie ist.

## Revendications

1. Utilisation d'une composition dans la préparation d'un médicament pour inhiber l'athérosclérose ou la resténose dans le système cardiovasculaire d'un hôte mammifère, ladite composition comprenant :
une construction génétique comprenant un gène codant pour la synthétase de l'oxyde nitrique dans un vecteur d'expression pour la transfection de cellules vasculaires ; dans laquelle ladite synthétase d'oxyde nitrique est exprimée par lesdites cellules vasculaires au niveau d'un site de lésion pour stimuler le niveau d'oxyde nitrique et inhiber l'hyperplasie myointimale.

2. Utilisation selon la revendication 1, dans laquelle ladite synthétase de l'oxyde nitrique est la synthétase de l'oxyde nitrique des cellules endothéliales.

3. Utilisation selon l'une quelconque des revendications 1 ou 2, dans laquelle ledit médicament est formulé pour être introduit dans ledit hôte sous forme de greffe ensemencée avec lesdites cellules vasculaires.

4. Utilisation selon l'une quelconque des revendications 1 à 3, dans laquelle lesdites cellules vasculaires sont des cellules endothéliales.

5. Utilisation selon l'une quelconque des revendications 1 à 3, dans laquelle lesdites cellules vasculaires sont des cellules de muscle vasculaire lisse.

6. Utilisation selon l'une quelconque des revendications 1 à 5, dans laquelle ladite préparation d'un médicament comprend le mélange de ladite construction génétique avec un liposome.

7. Utilisation selon l'une quelconque des revendications 1 à 6, dans laquelle ledit vecteur est un vecteur plasmide.

8. Utilisation selon l'une quelconque des revendications 1 à 6, dans laquelle ledit vecteur est un vecteur viral.

9. Utilisation selon l'une quelconque des revendications 1 à 8, dans laquelle ladite hyperplasie myointimale est consécutive à une angioplastie par ballonnet.
